# EUROPÄISCHE PATENTSCHRIFT

(11) **EP 0 959 068 B1**
(45) Veröffentlichungstag und Bekanntmachung des Hinweises auf die Patenterteilung: **15.12.2004**
(21) Anmeldenummer: 99108788.3
(22) Anmeldetag: 03.05.1999
(51) Int. Cl.: C07C 319/20, C07C 319/28, C07C 323/60

(54) **Verfahren zur Herstellung von wässrigen Natriummethioninat-Lösungen und Verwendung dieser Lösungen zur Herstellung eines Granulats**
Process for the preparation of aqueous sodium methioninate solutions and use of these solutions for the preparation of a granulate
Procédé pour la préparation d'une solution aqueuse de méthioninate de sodium et l'utilisation de ces solutions pour la préparation d'un granulat

(30) Priorität: 16.05.1998 DE 19822099
(43) Veröffentlichungstag der Anmeldung: 24.11.1999
(73) Patentinhaber: Degussa AG, 40474 Düsseldorf (DE)
(72) Erfinder: Körfer, Martin, 63867 Johannesberg (DE); Rohland, Lutz, 63065 Offenbach (DE); Binder, Wolfram Dr., 63517 Rodenbach (DE); Hasselbach, Hans Joachim Dr., 63571 Gelnhausen (DE); Alt, Hans Christian, 63571 Gelnhausen (DE); Huthmacher, Klaus Dr., 63571 Gelnhausen (DE); Kniesel, Heidemarie Dr., 63768 Hösbach (DE)

(56) Entgegenhaltungen:
- DE-A- 3 105 007

## Beschreibung

Die Erfindung betrifft ein Verfahren zur Herstellung von wäßrigen Natriummethioninat-Lösungen mit geringem Gehalt an Natriumcarbonat aus den bei der Verseifung von 5-(β-Methylmercaptoethyl)-hydantoin mit 1,1 bis 6 Äquivalenten Natriumhydroxid und/oder Natriumcarbonat anfallenden rohen Hydrolysegemischen durch Abtrennung von Natriumcarbonat-Monohydrat in der Hitze und durch verschiedene Verfahren anschließend aus diesen Lösungen hergestellte Granulate.

Methionin sowie wäßrige Lösungen von Methioninsalzen, insbesondere von Natriummethioninat (DE 31 05 009 C), aber auch Ersatzstoffe wie das Methionin-Hydroxyanaloge (MHA) werden weltweit als Futtermittelzusatz für die Aufzucht von Geflügel, Schweinen und anderen Nutztieren verwendet und kommen hauptsächlich der Produktion von tierischem Eiweiß zugute.

Je nach Erfordernissen werden Feststoff oder Flüssigformen bevorzugt eingesetzt.

Die im Handel befindliche Natriummethioninat-Lösung hat eine Konzentration von 40 Gew.% Methionin und entspricht im Gegensatz zum MHA hinsichtlich ihrer biologischen Wertigkeit dem festen Methionin, verglichen auf äquimolarer Basis. Für die Herstellung solcher Natriummethioninat-Lösungen kommen mehrere Methoden in Betracht z. B.
1. Auflösen von isoliertem Methionin in Natronlauge.
2. Alkalische Hydrolyse von 5-(β-Methylmercaptoethyl)-hydantoin mit NaOH und/oder Na₂CO₃, bzw. einem Gemisch von NaOH /Na₂CO₃/NaHCO₃.
3. Alkalische Hydrolyse von Methioninamid.

Verfahren 1 liefert zwar die reinste Produktform, ist aber durch einen zusätzlichen Arbeitsschritt im Vergleich zur Feststoffproduktion aufwendiger und damit weniger wirtschaftlich als die Herstellung von Methionin selbst. Dagegen setzen die Verfahren 2 bzw. 3 zu einem früheren Zeitpunkt der Methioninherstellung ein und erreichen damit eine kapazitätsmäßige Entlastung des Feststoffteils der DL-Methionin-Herstellung.

Die Herstellung von 5-(β-Methylmercaptoethyl)-hydantoin erfolgt in bekannter Weise durch Direktsynthese aus den üblichen Ausgangsstoffen Methylmercaptopropionaldehyd (MMP) und Blausäure in Gegenwart von Ammoniak und Kohlendioxid. Methioninamid stellt man in bekannter Weise durch Hydrolyse von Methioninnitril her, welches wiederum durch Direktsynthese aus den üblichen Ausgangsstoffen MMP, Blausäure oder Ammoniumcyanid und Ammoniak gewonnen wird.

Die nach dem Verfahren 2 anfallenden Verseifungslösungen enthalten größere Mengen an Natriumcarbonat, das abgetrennt werden muß.

Zu diesem Zweck konzentriert man gemäß DE-OS 31 04 997 das Hydrolysegemisch auf einen Gehalt von 40 bis 65 Gew.% Natriummethioninat auf, kühlt dann auf Raumtemperatur oder noch tiefer ab und trennt das dann ausfallende Natriumcarbonat-Dekahydrat ab. Dieses fällt jedoch häufig in nur schwer filtrierbarer Form an.

Gemäß US-PS 4 931 987 geht man umgekehrt vor. Man läßt zuerst das Natriumcarbonat auskristallisieren und konzentriert die entstehenden Lösungen anschließend auf.

Aus der DE-OS 31 05 009 ist ein Verfahren bekannt, bei dem man vor der Abtrennung Methanol oder Ethanol zusetzt.

In Abhängigkeit von speziellen Erfordernissen kann es zweckmäßig sein, eine feste oder flüssige Form des Tierfuttermittelsupplements einzusetzen.

Die Entscheidung für die Darreichungsform ist u. a. abhängig von den zur Verfügung stehenden Mischwerkzeugen bzw. den speziellen Präferenzen des jeweiligen Betreibers.

Bei der Herstellung des Mischfutters liegen zunächst die verschiedenen Futtermittel und Zusatzstoffe als Einzelkomponenten vor, die je nach Beschaffenheit z. B. durch Vermahlen, Verschroten, Trocknen oder Reinigen vorbereitet werden. Haben die einzelnen Komponenten die notwendige Beschaffenheit, wird der eigentliche Mischvorgang in einer dafür geeigneten Mischanlage vorgenommen. Die einzelnen Mischpartien sind dabei je nach Größe der Anlage verschieden. Die essentielle Aminosäure Methionin wird bei der Supplementierung von Mischfuttern in Konzentrationen in Größenordnungen von 0,01 bis 1,0 Gew.% eingesetzt. Diese Mengen werden durch entsprechende Wäge- und Dosiersysteme direkt dem Mischfutter zugesetzt.

In der DE-OS 31 05 009 wird beschrieben, daß wäßrige Lösungen von Natrium- oder Kaliummethioninat bei ihrer Verwendung als Futtermittelzusatz die gleiche Methioninwirksamkeit aufweisen wie festes Methionin.

Aus der DE-197 07 380 vom 15.02.1997 ist ein Granulat auf Methioninsalzbasis bekannt.

Aufgabe der Erfindung ist es, ein Verfahren zur Verfügung zu stellen, das wahlweise zu Natriumcarbonat und NaHCO₃armen oder auch -freien Natriummethioninat-Lösungen und den daraus herstellbaren Granulaten führt.

Gegenstand der Erfindung ist ein Verfahren zur Herstellung von wäßrigen Natriummethioninat-Lösungen mit einem geringen Gehalt an Natriumcarbonat und NaHCO₃ aus den bei der Verseifung von 5-(β-Methylmercaptoethyl)-hydantoin mit 1,1 bis 6 Äquivalenten Natriumhydroxid und/oder Natriumcarbonat anfallenden rohen Hydrolysegemischen, dadurch gekennzeichnet, daß man aus dem rohen Hydrolysegemisch , bevorzugt in zwei Stufen Wasser abdestilliert insbesondere nach dem Zusatz von weiterem Natriumhydroxid, bevorzugt bis zu einem Überschuß von 1 Mol/l, bezogen auf das Methioninat, bis der Natriummethioninat-Gehalt 60 bis 90 Gew.% beträgt und bei Temperaturen von 90 bis 140 ° C, insbesondere 110 bis 130 ° C, das ausgefallene Natriumcarbonat bzw. Bicarbonat abtrennt. Als Kristallisationsverfahren erweist sich die Verdampfungskristallisation als besonders geeignet.

Dabei werden die Filtrationseigenschaften durch die im heißen Zustand reduzierte Viskosität der Lösung sehr günstig beeinflußt.

Das Filtrat besitzt anschließend einen Rest-Natriumcarbonatgehalt von <6 Gew.%, bevorzugt ≤3,5 Gew.-%, insbesondere <1,5 Gew.-%.

Der im allgemeinen mit einer Zentrifuge oder Dekanter abgetrennte Feststoff, bestehend aus ca. 70 Gew.% Natriumcarbonat und ca. 15 Gew.% Methionin, fällt erfindungsgemäß im Gegensatz zum Stand der Technik bei hoher Temperatur an. Er wird dann ohne Abkühlung in die bei Temperaturen oberhalb von 120 ° C ablaufende Hydrolyse des Hydantoins zurückgeführt. Durch dieses Vorgehen ist ein wirtschaftliches Kreislaufverfahren zur Produktion der Na-Methioninatlösung realisierbar.

Ist der noch vorhandene restliche Gehalt an Natriumcarbonat bzw. Hydrogencarbonat in der Lösung unerwünscht, wird er durch eine dem Natriumionen-Gehalt entsprechende molare Zugabe an freiem Methionin reduziert. Das Methionin kann sowohl in Form
einer Lösung oder als Suspension zugesetzt werden. Die Produktendkonzentration stellt man gegebenenfalls durch den Zusatz von Wasser ein.

Durch thermische Behandlung bei Temperaturen von ca. 80 bis 200 ° C, insbesondere 130 bis 170 ° C, Verweilzeiten zwischen 0.1 und 3 Stunden kann anschließend das Carbonat unter CO₂-Abspaltung zersetzt und Na-Methioninat gebildet werden. Zum gezielten Austrag des CO₂ aus der Lösung wird ein inertes Treibmittel oder Wasserdampf eingesetzt. Sofern als Treibmittel Wasserdampf eingesetzt wird, kann dieser sowohl extern zugeführt werden als auch mit geeigneten Apparaten wie z. B. Fallfilmverdampfern aus der Lösung ausgedampft werden.

Der Austrag des CO₂ kann weiter unterstützt werden durch Einsatz von mechanischer Energie z. B. in Form von Rühren oder durch gezielten Einsatz von verfahrenstechnischen Apparaten, welche insbesondere die Durchführung chemischer Reaktionen mit überlagertem Stoffübergang von Flüssigphase zu Gasphase begünstigen. Dazu eignen sich z. B. Gegenstrombodenkolonnen, Füllkörperkolonnen, Jetapparate oder Blasensäulen.

Nach diesem Vorgehen werden besonders lagerstabile Lösungen des Na-Methioninats erhalten. Weitere Gegenstände der Erfindung sind aus den so hergestellten Natriumcarbonatfreien oder -armen Lösungen durch formgebende Maßnahmen hergestellte rieselfähige Granulate.

In einer vorteilhaften Variante wird die Lösung unter atmosphärischem oder reduziertem Druck weiter auf konzentriert, so daß der Wasserentzug aus der Lösung durch die energetisch sehr günstige
reine Verdampfung und nicht durch Trocknung eintritt. Bei Drücken von 20 bis 1000 mBar und Temperaturen zwischen 100 und 160 °C wird dabei eine noch frei fließfähige Lösung oder Pseudoschmelze erhalten. Diese Lösung hat dann nur noch Restwassergehalte von ca. 0,5 bis 3 Gew.%. Diese Pseudoschmelze hat dann eine Erstarrungstemperatur von ca. 40 bis 80°C.

Aufgrund dieser günstigen Randbedingungen sind dann Wirbelschichtgranulationsverfahren besonders geeignet, um Granulate zu erzeugen, wie sie im Produktanwendungsbereich eingesetzt werden.

Das Granulat besitzt eine Schüttdichte von > 650 kg/m³, bevorzugt > 700 kg/m³, bei einer Korngrößenverteilung von 63 bis 5000 µm, bevorzugt 100 bis 3000 µm, besonders bevorzugt 100 bis 1400 µm, wobei ≈ 90 % in einer Korngröße > 100 µm vorliegen.

Der Anteil mit einer Korngröße <63 µm liegt im allgemeinen bei maximal 2 %, bevorzugt 1 %, der Staubanteil nach Dr. Groschopp bei 1 %, bevorzugt <0,5 %.

Ein besonders geeigneter Granulationsprozess für Lösungen mit Natriummethioninatgehalten >65 Gew.% bis 90 Gew.% arbeitet 3-stufig, einem Granulaterzeuger, einem Trockner und einem Produktkühler.
Im einzelnen gilt:
a) als Granulation ist besonders die Wirbelschicht-Aufbaugranulation geeignet,
b) die Trocknung kann sowohl in der Wirbelschicht als auch in z. B. einem Vakuumkontaktrockner durchgeführt werden
c) für die Kühlung kommen ebenfalls z. B. Wirbelschicht, Kühlschnecke oder Kühlrinne zum Einsatz.

Als Trocknungsgas dient vorgetrocknete, erhitzte Luft oder Stickstoff; der Taupunkt des Gases liegt bei ca. -10 bis 40 °C. Die Granulaterzeugung findet durch Versprühen der hochkonzentrierten Produktlösung direkt in die Wirbelschicht statt. Als Zerstäubungsaggregat kann, anders als in der Technik üblich, eine als Druckdüse betriebene Zweistoffdüse Verwendung finden. Dabei wird die Tropfenerzeugung durch den spontanen Druckabfall am Düsenkopf durchgeführt; die Mantelluft kann günstigerweise gegenüber der üblichen Betriebsweise (Massenverhältnis Lösung : Luft ca 2 : 1) auf einen deutlich geringeren Wert von 7 : 1 bis 10 : 1 reduziert werden. Die Mantelluft dient zum Eintrag der Tropfen in die Wirbelschicht.

Von besonderer Bedeutung für die Temperaturführung des Apparates sind die Luftzustände in Verbindung mit der Produktfeüchte in der Wirbelschicht. Die Korngröße der Granulate kann im wesentli.hen über die Erzeugung und den Eintrag von Granulatkeimen durch intern in die Wirbelschicht eingebaute Brechwerkzeuge, z. B. Stiftmühle oder externe Vermahlung, z. B. von Überkorn aus der Wirbelschicht vorgenommen werden.

Der Trocknungsschritt, kann sich direkt an die Granulation im gleichen Apparat anschließen. Durch geeignete Trennelemente sowohl an der Trocknungszufuhr als auch in der Produktseite muß eine unabhängige Temperaturführung möglich sein. Das mit dem Trocknungsgas ausgetragene Feinkorn bzw. der Abrieb kann ebenfalls in die Granulationsstufe zurückgeführt werden. Die Temperatur in der Trocknungsstufe ist ebenfalls von der Feuchte des Trocknungsgases und des Granulats abhängig und liegt im Bereich zwischen 80 und 120 °C.

Bei der Kühlung auf im allgemeinen 30 bis 60 °C muß sichergestellt sein, daß das Produkt bis zum Erkalten mechanisch in einer trockenen Atmosphäre bewegt und trockengehalten wird, um ein Verkleben zu verhindern. Aus diesem Grund sind Wirbelschichtkühler oder Kühlschnecken oder Kühlrinnen als sehr geeignete Apparate anzusehen. Auch können andere mechanisch schonende Apparate eingesetzt werden, z. B. Kühlrinnen, Tellerkühler oder Kühlschnecken. Diese Apparate sind zumindest mit einem trockenen Gas zu Überlagern um ein Produktverkleben zu vermeiden.

Das gesamte Wirbelschichtaufbaugranulationsverfahren kann nicht nur kontinuierlich sondern ebenfalls batchweise in nur einer einzigen Kammer einer Wirbelschicht durchgeführt werden. Dabei erfolgt die Aufbaugranulation, Trocknung und Kühlung in zeitlicher Abfolge durch entsprechende Temperaturführung.

Alternativ zum geschilderten Granulaterzeugungsverfahren durch Wirbelschichtaufbaugranulation bietet sich zur Granulaterzeugung das Verdüsen mit Zweistoff- oder Glattstrahldüsen oder ein Vertropfungsverfahren an. Dabei wird die höchstkonzentrierte Lösung bzw. Pseudoschmelze mit einer Restfeuchte von 0,5 bis 10 Gew.-% bei Temperaturen zwischen 70 ° C, bevorzugt 120 und 160 °C durch eine Lochplatte mit feinen Bohrungen gedrückt. Durch Anregung dieser Platte in axiale Schwingungen kann ein besonders einheitliches Tropfenspektrum auch bei dieser hochviskosen Lösung erhalten werden. Erfindungsgemäß läßt man diese Tropfen in einem Fallturm in getrocknetem Inertgas, z. B. Luft oder Stickstoff erstarren.

Die Erstarrung wird dabei bei leicht erhöhten Temperaturen durchgeführt, so daß gleichzeitig eine weitere Wasserverdunstung stattfindet. Abschließend werden die Kugeln in einer Wirbelschicht aufgefangen und unter den oben geschilderten Bedingungen nachgetrocknet. Die so erhaltenen Granulatkugeln zeichnen sich durch eine besonders gute Rieselfähigkeit, einheitliches Kornspektrum und Staubarmut aus.

Eine weitere Variante stellt die Herstellung des Granulats durch Extrusion der Pseudoschmelze der hochkonzentrierten Lösung in einem Extruder mit gezielter Temperaturführung dar. Dabei kann ohne weitere Zusatzstoffe direkt ein Extrudat erzeugt werden. Je nach eingesetzten Matrizen lassen sich Granulate von ca. 500 bis 5000 µm Durchmesser erzeugen. Sofern gewünscht und notwendig, kann das Produkt ebenfalls abschließend nachgetrocknet werden.

Zur Verbesserung der Handhabbarkeit der herzustellenden Granulate ist es empfehlenswert, die Methioninsalzlösungen in Gegenwart von Zuschlagstoffen auf silikatischer Basis zu versprühen und zu granulieren.

Zu diesen gehören hydrophile und hydrophobe Kieselsäuren, pyrogener Natur oder durch Fällung hergestellte von 5 bis 300 m³/g, vorzugsweise sprühgetrocknete Kieselsäuren. Verwendbar sind auch feinteilige Zeolithe, z. B. des Typs A oder Bentonite.

Dieser Zuschlagstoffe kann man entweder in der zu versprühenden Lösung suspendieren oder bevorzugt mit dem Luftstrom in die Apparatur eindosieren, in der die Lösung versprüht und gegebenenfalls granuliert wird.

Die Menge weiterer Zuschlagstoffe beläuft sich auf 0,1 bis 10 Gew.%, bevorzugt 0,1 bis 5 Gew.%, bezogen auf den granulierten Feststoff. Zu diesen Zuschlagstoffen gehören neben den silikatischen Verbindungen naturgemäß bevorzugt Substanzen, die im Futtermittelbereich zugelassen sind, insbesondere Fettsäuren und deren Salze, bevorzugt Alkalioder Erdalkalisalze.

Zu den Fettsäuren gehören insbesondere Stearinsäure und Palmitinsäure bzw. Mischungen von C₁₆ bis C₁₈-Kohlenstoffatomen enthaltenden Fettsäuren oder deren obengenannten Salzen.

Während das nicht granulierte Natriummethioninat aufgrund seines hygroskopischen Verhaltens leicht verklumpt und seine Rieselfähigkeit verliert, findet man dies bei den erfindungsgemäßen Granulaten unerwarteterweise nicht. Sie bleiben auch bei Bewitterung rieselfähig und leicht handhabbar.

### Beispiele

### Beispiel 1

Es werden 1000 g einer Lösung eingesetzt, welche durch Lösen von 200 g Methionin und einer äquimolaren Menge NaOH unter Zusatz von 80 g Na₂CO₃ in 666 g Wasser erhalten wird. Dieser Lösung wird unter Rühren eine Menge von 600 g Wsser durch Destillation entzogen und der entstandenen Niederschlag in einer Siebzentrifuge bei Temperaturen >100 ° C abgetrennt. Das Filtrat enthält 71 % Na-Methioninat und nur 1,1 % Na₂CO₃, NaHCO₃; als Na₂CO₃ gerechnet.

### Beispiel 2

Es werden 1000 g eines rohen Hydrolysegemisches eingesetzt, welches durch Verseifung einer wäßrigen Lösung von 5-(β-Methylmercapto-ethyl)-hydantoin mit einer 2,5 mol äquivalenten wäßrigen Na⁺-Lösung als einem Gemisch von NaOH, Na₂CO₃ und NaHCO₃ bei 160 bis 180 ° C unter Entfernung von CO₂ und NH₃ aus dem Reaktionsgemisch hergestellt wurde, und z. B. 231 g Na-Methioninat und 84 g eines Gemisches von Na₂CO₃ und NaHCO₃, gerechnet als Na₂CO₃ enthält. Aus diesem rohen Hydrolysegemisch werden 550 g Wasser abdestilliert und der gebildete Niederschlag bei Siedetemperaturen von 127 ° C über ein beheiztes Druckfilter abgetrennt. Das Filtrat enthält 60 % Na-Methioninat und 5,4 % eines Gemisches von Na₂CO₃, NaHCO₃; als Na₂CO₃ gerechnet

### Beispiel 3

Das Beispiel 1 wird wiederholt mit dem Unterschied, daß die Eindampfung in zwei Stufen erfolgt. In der ersten Stufe werden 350 g Wasser abdestilliert, die Suspension wird dann eine gewisse Zeit in diesem Zustand gerührt, anschließend werden nochmals 200 g Wasser abdestilliert. Der Niederschlag wird wiederum bei Siedetemperaturen von 127 ° C über ein beheiztes Druckfilter abgetrennt. Das Filtrat enthält 62 % Natriummethioninat und nur 3,2 % Na₂CO₃, NaHCO₃; als Na₂CO₃ gerechnet.

### Beispiel 4

Das Beispiel 2 wird wiederholt mit dem Unterschied, daß in der zweiten Stufe 250 g Wasser abdestilliert werden. In diesem Fall liegt die Siedetemperatur bei 131 ° C, das Filtrat enthält 71 % Natriummethioninat und nur 3,5 % Na₂CO₃ NaHCO₃; als Na₂CO₃ gerechnet.

### Beispiel 5

Es werden 1000 g Lösung einsetzt, wie sie gemäß Beispiel 3 als Filtrat erhalten wird, um mit einer Temperatur von 85 bis 130 ° C in ein Wirbelbett von bereits getrocknetem Granulat eingedüst zu werden. Die Tropen der Lösung verteilen sich als dünne Schicht auf dem vorgelegten Granulat und werden von 6 Nm³ N₂ getrocknet, wobei der Stickstoff in der verwendeten Apparatur von 95 ° C auf 58 ° C abkühlt und sich der Taupunkt des Gases von 8 ° C auf 35 ° C erhöht. Die Restfeuchte des Granulats beträgt 6,4 %.

### Beispiel 6

Das Beispiel 4 wird wiederholt mit dem Unterschied, daß das Trocknungsgas vor Eintritt in die Wirbelschicht auf einen Taupunkt von z. B. -2 ° C entfeuchtet wird. Mit 15 Nm³ N₂ hat das Granulat eine Restfeuchte von nur 2, 4 %.

## Patentansprüche

1. Verfahren zur Herstellung von wäßrigen Natriummethioninat-Lösungen mit einem geringen Gehalt an Natriumcarbonat aus den bei der Verseifung von 5-(β-Methylmercapto-ethyl)-hydantoin mit 1,1 bis 6 Äquivalenten Natriumhydroxid und/oder Natriumcarbonat anfallenden rohen Hydrolysegemischen,
**dadurch gekennzeichnet,**
**daß** man aus dem rohen Hydrolysegemisch Wasser abdestilliert, bis der Natriummethioninat-Gehalt 60 bis 90 Gew.% beträgt, und bei einer Temperatur von 90 bis 140 ° C das ausgefallene Natriumcarbonat, wasserfrei als Monohydrat und/oder Bicarbonat abtrennt.

2. Verfahren gemäß Anspruch 1 **dadurch gekennzeichnet, daß** man aus dem Hydrolysegemisch Wasser abdestiliert, nach dem Zusatz weiteren Natrium hydroxids,

3. Verfahren gemäß Anspruch 1,
**dadurch gekennzeichnet,**
**daß** man das abgetrennte, Natriummethioninatreste enthaltende Natriumcarbonat (wasserfrei, Monohydrat und/oder Bicarbonat), ohne es abzukühlen, recycliert und in die Verseifungsstufe zurückführt.

4. Verfahren gemäß Anspruch 1,
**dadurch gekennzeichnet,**
**daß** man der Lösung nach der Abtrennung des Natrium carbonats, wasserfrei, als Monohydrat und/oder Bicarbonat freies Methionin in einer Menge zusetzt,
die der des restlichen Natriumcarbonats molar in etwa äquivalent ist, die Lösung auf 80 bis 200 °C,
gegebenenfalls unter Druck, erhitzt und das entstehende CO₂ aus der Lösung austrägt.

5. Verfahren gemäß Anspruch 3,
**dadurch gekennzeichnet,**
**daß** man zum Austrag des CO₂ Wasserdampf oder ein inertes Gas einsetzt.

6. Verfahren gemäß Anspruch 3,
**dadurch gekennzeichnet,**
**daß** man zum Austrag von CO₂ die Lösung rührt.

7. Verfahren gemäß einem oder mehreren vorhergehenden Ansprüche,
**dadurch gekennzeichnet,**
**daß** man die Natriumcarbonat-arme oder -freie Lösung anschließend in ein rieselfähiges Granulat überführt.

8. Verfahren gemäß Anspruch 6,
**dadurch gekennzeichnet,**
**daß** man eine Wirbelschichtgranulation einsetzt.

9. Verfahren gemäß Anspruch 7,
**dadurch gekennzeichnet,**
**daß** man die Natriumcarbonat-arme oder -freie Lösung bei Temperaturen von 100 bis 160 ° C und Drucken von 10 bis 1000 mbar bis auf einen Restwassergehalt von 0,5 bis 3 Gew.% aufkonzentriert und dann einer Wirbelschichtgranulation unterzieht.

10. Verfahren gemäß Anspruch 6,
**dadurch gekennzeichnet,**
**daß** man die Natriumcarbonat-arme oder -freie Lösung einer Aufbauwirbelschichtgranulation unterwirft.

11. Verfahren gemäß Anspruch 9,
**dadurch gekennzeichnet,**
**daß** man eine Lösung mit einem Gehalt >65 Gew.% bis 95 Gew.% Natriummethinoninat einsetzt.

12. Verfahren gemäß den Ansprüchen 9 und 10,
**dadurch gekennzeichnet,**
**daß** der Granulatprozeß in drei Stufen
a) die Granulaterzeugung
b) die Trocknung
c) die Granulatkühlung
unterteilt ist.

13. Verfahren gemäß den Ansprüchen 9 bis 11,
**dadurch gekennzeichnet,**
**daß** das Masseverhältnis von Lösung : Luft (Mantelluft) zwischen 7 : 1 und 10 : 1 liegt.

14. Verfahren gemäß Anspruch 9,
**dadurch gekennzeichnet,**
**daß** man Natriummethioninat mit einem Gehalt von 0,5 bis 10 Gew.% an Restfeuchte bei 70 bis 160 ° C durch eine Lochplatte mit feinen Bohrungen drückt und diese Platte gegebenfalls in axiale Schwingungen versetzt (Vertropfungsverfahren)oder verdüst, d. h. zerstäubt, zerwellt, zertropft und das erhaltene Granulat gegebenenfalls anschließend trocknet.

15. Verfahren gemäß Anspruch 8,
**dadurch gekennzeichnet,**
**daß** man eine Lösung mit einem Gehalt von 80 bis 99,5, vorzugsweise 93 bis 99,5 Gew.% an Natriummethioninat extrudiert und das Extrudat gegebenenfalls anschließend trocknet.

## Claims

1. Process for the preparation of aqueous sodium methioninate solutions having a low sodium carbonate content from the crude hydrolysis mixtures obtained in the hydrolysis of 5-(β-methylmercaptoethyl)-hydantoin with from 1.1 to 6 equivalents of sodium hydroxide and/or sodium carbonate,
**characterised in that**
water is distilled off from the crude hydrolysis mixture, until the sodium methioninate content is from 60 to 90 wt.%, and the resulting sodium carbonate is separated off, in anhydrous form as the monohydrate and/or hydrogen carbonate, at a temperature of from 90 to 140°C.

2. Process according to claim 1,
**characterised in that**
water is distilled off from the hydrolysis mixture after the addition of further sodium hydroxide.

3. Process according to claim 1,
**characterised in that**
the sodium carbonate (anhydrous, monohydrate and/or hydrogen carbonate) containing sodium methioninate residues that has been separated off is recycled, without being cooled, and returned to the hydrolysis stage.

4. Process according to claim 1,
**characterised in that**
after the sodium carbonate has been separated off, in anhydrous form as the monohydrate and/or hydrogen carbonate, free methionine is added to the solution in an amount that is approximately the molar equivalent of the amount of residual sodium carbonate, the solution is heated to from 80 to 200°C, optionally under pressure, and the CO₂ that forms is removed from the solution.

5. Process according to claim 3,
**characterised in that**
water vapour or an inert gas is used for removing the CO₂.

6. Process according to claim 3,
**characterised in that**
the solution is stirred to remove CO₂.

7. Process according to one or more of the preceding claims,
**characterised in that**
the solution containing no or only a small amount of sodium carbonate is then converted into a pourable granulate.

8. Process according to claim 6,
**characterised in that**
fluidised-bed granulation is employed.

9. Process according to claim 7,
**characterised in that**
the solution containing no or only a small amount of sodium carbonate is concentrated at temperatures of from 100 to 160°C and pressures of from 10 to 1000 mbar to a residual water content of from 0.5 to 3 wt.% and is then subjected to fluidised-bed granulation.

10. Process according to claim 6,
**characterised in that**
the solution containing no or only a small amount of sodium carbonate is subjected to build-up fluidised-bed granulation.

11. Process according to claim 9,
**characterised in that**
a solution containing from > 65 wt.% to 95 wt.% sodium methioninate is used.

12. Process according to claims 9 and 10,
**characterised in that**
the granulation process is divided into three stages
a) production of the granulate
b) drying
c) cooling of the granulate.

13. Process according to claims 9 to 11,
**characterised in that**
the weight ratio of solution : air (enveloping air) is from 7 : 1 to 10 : 1.

14. Process according to claim 9,
**characterised in that**
sodium methioninate having a residual moisture content of from 0.5 to 10 wt.% is pressed at from 70 to 160°C through a perforated plate having fine holes and the plate is optionally made to vibrate axially (droplet-forming process) or atomised, i.e. sprayed, subjected to wavy sheet disintegration, formed into droplets, and the resulting granulate is then optionally dried.

15. Process according to claim 8,
**characterised in that**
a solution containing from 80 to 99.5 wt.%, preferably from 93 to 99.5 wt.%, of sodium methioninate is extruded and the extrudate is then optionally dried.

## Revendications

1. Procédé pour la préparation de solutions aqueuses de méthionate de sodium présentant une faible teneur en carbonate de sodium à partir des mélanges d'hydrolyse bruts produits lors de la saponification de 5-(β-méthylmercaptoéthyl)hydantoïne avec 1,1 à 6 équivalents d'hydroxyde de sodium et/ou de carbonate de sodium, **caractérisé en ce qu'**on élimine par distillation de l'eau du mélange d'hydrolyse brut jusqu'à ce que la teneur en méthionate de sodium soit comprise entre 60 et 90% en poids et on sépare à une température de 90 à 140°C le carbonate de sodium précipité, sous forme anhydre, de monohydrate et/ou de bicarbonate.

2. Procédé selon la revendication 1, **caractérisé en ce qu'**on élimine de l'eau du mélange d'hydrolyse après addition d'hydroxyde de sodium supplémentaire.

3. Procédé selon la revendication 1, **caractérisé en ce qu'**on recycle le carbonate de sodium (anhydre, monohydraté et/ou le bicarbonate) séparé, contenant des restes de méthionate de sodium, sans le refroidir et on le réalimente dans l'étape de saponification.

4. Procédé selon la revendication 1, **caractérisé en ce qu'**on ajoute à la solution, après la séparation du carbonate de sodium, sous forme anhydre, de monohydrate et/ou de bicarbonate, de la méthionine libre en une quantité qui est environ' équivalente en moles au carbonate de sodium résiduel, on chauffe la solution à 80 jusqu'à 200°C, le cas échéant sous pression et on évacue le CO₂ formé de la solution.

5. Procédé selon la revendication 3, **caractérisé en ce qu'**on utilise de la vapeur d'eau ou un gaz inerte pour l'évacuation du CO₂.

6. Procédé selon la revendication 3, **caractérisé en ce qu'**on agite la solution pour l'évacuation du CO₂.

7. Procédé selon l'une ou plusieurs des revendications précédentes, **caractérisé en ce qu'**on transforme ensuite la solution pauvre en carbonate de sodium ou exempte de carbonate de sodium en un granulat pouvant s'écouler.

8. Procédé selon la revendication 6, **caractérisé en ce qu'**on utilise une granulation en lit fluidisé.

9. Procédé selon la revendication 7, **caractérisé en ce qu'**on concentre la solution pauvre en carbonate de sodium ou exempte de carbonate de sodium à des températures de 100 à 160°C et des pressions de 10 à 1000 mbars jusqu'à une teneur en eau résiduelle de 0,5 à 3% en poids, puis on la soumet à une granulation en lit fluidisé.

10. Procédé selon la revendication 6, **caractérisé en ce qu'**on soumet la solution pauvre en carbonate de sodium ou exempte de carbonate de sodium à une granulation d'élaboration en lit fluidisé.

11. Procédé selon la revendication 9, **caractérisé en ce qu'**on utilise une solution présentant une teneur en méthionate de sodium >65% en poids jusqu'à 95% en poids.

12. Procédé selon les revendications 9 et 10, **caractérisé en ce que** le processus de granulation est divisé en trois étapes
a) la production du granulat
b) le séchage
c) le refroidissement du granulat.

13. Procédé selon les revendications 9 à 11, **caractérisé en ce que** le rapport massique solution:air (air enveloppant) est compris entre 7 : 1 et 10 : 1.

14. Procédé selon la revendication 9, **caractérisé en ce qu'**on comprime le méthionate de sodium, présentant une teneur en humidité résiduelle de 0,5 à 10% en poids à 70 jusqu'à 160°C à travers une plaque perforée avec des petits trous et on déplace cette plaque le cas échéant par des oscillations axiales (procédé dé formation de gouttes) ou on l'atomise, c'est-à-dire qu'on le pulvérise, on le désagrège, on le transforme en gouttes et on sèche ensuite le granulat obtenu.

15. Procédé selon la revendication 8, **caractérisé en ce qu'**on extrude une solution présentant une teneur en méthionate de sodium de 80 à 99,5, de préférence de 93 à 99,5% en poids et on sèche ensuite le cas échéant l'extrudat.
